(19) Europäisches Patentamt
European Patent Office
Office européen des brevets

(11) Veröffentlichungsnummer: **0 052 599**
**B1**

(12) **EUROPÄISCHE PATENTSCHRIFT**

(45) Veröffentlichungstag der Patentschrift: **22.05.85**

(51) Int. Cl.⁴: **C 07 C 49/835,** C 07 C 45/68, C 07 C 143/68 // C07C49/813, C07C45/63, C07C83/06, C07D223/16

(21) Anmeldenummer: **82100359.7**

(22) Anmeldetag: **04.02.80**

(60) Veröffentlichungsnummer der früheren Anmeldung nach Art. 76 EPÜ:

(54) **Propargylderivate und deren Herstellung.**

(30) Priorität: **07.02.79 US 10118**
**22.01.80 CH 511/80**

(43) Veröffentlichungstag der Anmeldung: **26.05.82 Patentblatt 82/21**

(45) Bekanntmachung des Hinweises auf die Patenterteilung: **22.05.85 Patentblatt 85/21**

(84) Benannte Vertragsstaaten: **AT BE CH DE FR GB IT LU NL SE**

(56) Entgegenhaltungen:
**EP - A - 0 030 015**
**FR - A - 2 278 327**

(73) Patentinhaber: **F. HOFFMANN-LA ROCHE & CO. Aktiengesellschaft, CH-4002 Basel (CH)**

(72) Erfinder: **Trybulski, Eugene J., 13 Homer Street, Parsippany, N.J. 07054 (US)**

(74) Vertreter: **Lederer, Franz, Dr. et al, Patentanwälte Dr. Lederer Franz Meyer-Roxlau Reiner F. Lucile-Grahn-Strasse 22, D-8000 München 80 (DE)**

EP 0 052 599 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die vorliegende Erfindung betrifft Propargylderivate der allgemeinen Formel

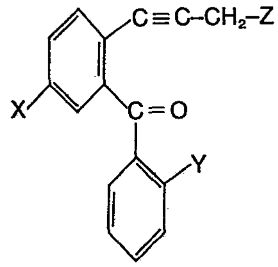

I

worin X und Y je Wasserstoff, Halogen mit einer Atomnummer von höchstens 35 oder Trifluormethyl und

Z Hydroxy, Methansulfonyloxy, Toluolsulfonyloxy, Brom oder Chlor bedeuten.

Die Verbindungen der Formel I sind nützlich als Zwischenprodukte bei der Herstellung von pharmakologisch aktiven 2-Benzazepinen. Sie können erfindungsgemäss dadurch hergestellt werden, dass man eine Verbindung der allgemeinen Formel

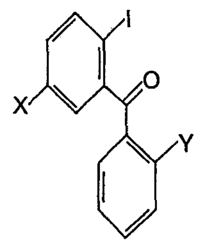

II

worin X und Y obige Bedeutung besitzen, in Gegenwart eines Palladium-(II)-Salzes, eines Organophosphins, eines tertiären oder sekundären Amins und von Kupfer-(I)-Jodid mit Propargylalkohol zur Reaktion bringt und erwünschtenfalls die erhaltene Verbindung der Formel I, worin Z Hydroxy bedeutet, in eine Verbindung der Formel I, worin Z Methansulfonyloxy, Toluolsulfonyloxy, Brom oder Chlor bedeutet, überführt.

In der vorliegenden Beschreibung bezeichnen die Ausdrücke «Halogen» oder «Halo» die drei Formen Chlor, Brom oder Fluor.

Durch das nachfolgende Reaktionsschema, worin X und Y obige Bedeutung haben, wird die vorliegende Erfindung näher illustriert:

Schema

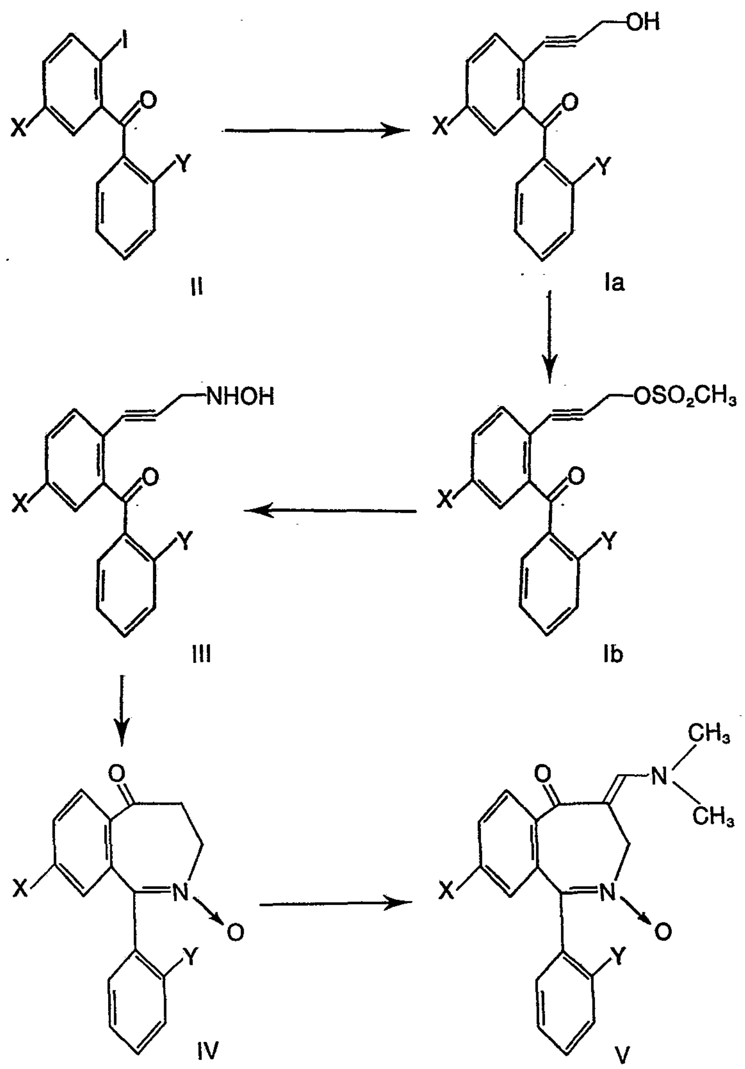

II Ia III Ib IV V

II→Ia

Die Verbindung der Formel Ia kann erhalten werden, indem man eine Verbindung der Formel II in Gegenwart eines Palladium-(II)-Salzes (wie Palladiumchlorid oder Palladiumacetat), eines Organophosphins (z.B. Triphenylphosphin), Kupfer-(I)-Jodid und eines tertiären oder vorzugsweise sekundären Amins (wie Diäthylamin oder Diisopropylamin) mit Propargylalkohol zur Reaktion bringt. Als Lösungsmittel für diese Reaktion kann das Amin selbst (z.B. Diäthylamin), ein halogenierter Kohlenwasserstoff (z.B. Methylenchlorid), Dimethylformamid oder ein ätherisches Lösungsmittel dienen. Die Reaktionstemperatur kann in einem Bereich von etwa 0 bis etwa 70 °C liegen, wobei Raumtemperatur bevorzugt ist. Die Anwesenheit von Kupfer-(I)-Jodid ist zwingend, wenn die Reaktion bei Raumtemperatur oder unterhalb davon durchgeführt wird, wogegen dies nicht der Fall ist, wenn die Reaktion unter Erhitzen durchgeführt wird. Die Anwesenheit des Organophosphins ist nicht absolut notwendig, jedoch höchst vorteilhaft. Anstelle von Palladium-(II)-Salz plus Organophosphin kann auch ein geeigneter Komplex verwendet werden, wie z.B. Dichlor-bis-(triphenylphosphin)-palladium-(II).

Die Ausgangsprodukte der Formel II können dadurch hergestellt werden, dass man ein entsprechendes bekanntes Aminobenzophenon mittels Natriumnitrit in Schwefelsäure diazotiert und die Salze durch Ausfällung der entsprechenden Tetrafluoroborate isoliert, worauf man diese Tetrafluoroborate in Wasser suspendiert und mit wässrigem Kaliumjodid behandelt, um so das Jodbenzophenon zu erhalten. Diese Reaktion werden unter Verwendung an sich bekannter Methoden durchgeführt.

Ia→Ib

Die Verbindung der Formel Ia kann in Gegenwart eines tertiären Amins, wie Triäthylamin, und in Gegenwart eines organischen Lösungsmittels, wie halogenierte Kohlenwasserstoffe (z.B. Methylenchlorid), Toluol oder Diäthyläther mit Methansulfonylchlorid umgesetzt werden. Die Reaktion kann zwischen etwa −78 °C und Raumtemperatur durchgeführt werden, wobei Temperaturen um 0 °C bevorzugt sind. Verbindungen entsprechend denjenigen der Formel Ib, welche jedoch als Abgangsgruppe nicht Methansulfonyloxy, sondern Toluolsulfonyloxy, Brom oder Chlor enthalten, können ebenfalls ausgehend von Verbindungen der Formel Ia nach an sich bekannten Methoden hergestellt werden, beispielsweise mittels Toluolsulfonylchlorid, Thionylchlorid, Phosphortribromid und dergleichen.

Ib→III→IV

Die Verbindung der Formel Ib oder ein Analogon davon, welches als Abgangsgruppe nicht Methansulfonyloxy, sondern Toluolsulfonyloxy, Brom oder Chlor enthält, wird mit Hydroxylamin zur Reaktion gebracht. Dies erfolgt zweckmässigerweise in einem Alkohol ($C_1$ bis $C_4$) als Lösungsmittel und bei einer Reaktionstemperatur von etwa 0 °C bis Raumtemperatur, wobei etwa Raumtemperatur bevorzugt ist. Das isolierte Zwischenprodukt III kann dann in Gegenwart einer Säure, beispielsweise einer Carbonsäure oder einer verdünnten oder konzentrierten Mineralsäure, und zweckmässigerweise in Gegenwart eines inerten organischen Lösungsmittels mit einem Quecksilber-(II)-Salz zur Reaktion gebracht werden, beispielsweise mit dem Sulfat, Chlorid, Acetat, Trifluoracetat oder einem Sulfonat (einschliesslich Salze mit Sulfonsäuregruppen enthaltenden Ionenaustauschern). So kann die Verbindung der Formel III in einem inerten Lösungsmittel, z.B. einem halogenierten Kohlenwasserstoff, wie Methylenchlorid, mit einer Mischung von Quecksilber-(II)-Sulfat und einer Carbonsäure ($C_1$ bis $C_5$), wie Ameisensäure oder Essigsäure, zur Reaktion gebracht werden. Die Reaktionstemperatur liegt zwischen etwa −10 °C und Raumtemperatur, vorzugsweise zwischen etwa 0 und 5 °C. Anderseits kann die Verbindung der Formel III auch in Gegenwart von Wasser mit einer starken Säure behandelt werden, beispielsweise mit konzentrierter Schwefelsäure bei etwa −10 °C bis Raumtemperatur, vorzugsweise bei etwa 0 °C.

IV→V

Die Verbindung der Formel IV kann dann in einem inerten Lösungsmittel, beispielsweise einem halogenierten Kohlenwasserstoff (wie Methylenchlorid), Dimethylformamid oder einem hochsiedenden Äther, mit Dimethylformamid-dimethylacetal zur Reaktion gebracht werden. Die Reaktionstemperatur kann zwischen etwa 0 und 100 °C liegen, wobei Raumtemperatur bevorzugt ist.

Die Verbindungen der Formel V sind nützliche Zwischenprodukte für die Herstellung von 2-Benzazepinen, beispielsweise von Pyrimido-2-benzazepinen der Formel

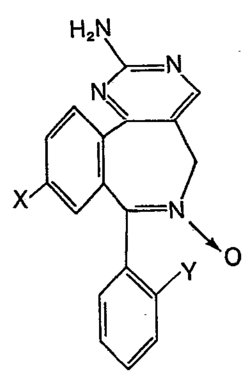

VI

worin X und Y obige Bedeutung besitzen.

Die Verbindung der obigen Formel VI kann dadurch hergestellt werden, dass man eine Verbindung der Formel V mit einer Verbindung der Formel

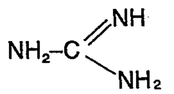

VII

zur Reaktion bringt. Hierfür kann irgendein inertes organisches Lösungsmittel verwendet werden, wie Methylenchlorid, Alkohole (wie Methanol), Äther (wie Dioxan, Tetrahydrofuran) oder Dimethylformamid; die Reaktionstemperatur kann zwischen etwa Raumtemperatur und Rückflusstemperatur des verwendeten Lösungsmittels liegen, wobei etwa Raumtemperatur bevorzugt ist.

Die obige Reaktionen von Ib zu V und von V mit VII zu VI sind nicht Gegenstand der vorliegenden Erfindung, sondern werden zur Vervollständigung der Offenbarung bezüglich der Verwendbarkeit der Verbindungen der Formel I beschrieben. Die Pyrimido-2-benzazepine der Formel VI entfalten anxiolytische und sedative Aktivitäten.

In den nachfolgenden Beispielen sind alle Temperaturen in Celsiusgraden angegeben. Beispiele 1 und 2 illustrieren die Herstellung von Ausgangsprodukten; Beispiele 3 bis 5 illustrieren die Herstellung und Beispiele 6 und 7 die Weiterverarbeitung von erfindungsgemässen Propargylderivaten.

Beispiel 1

Eine Mischung aus 76 g (1,1 Mol) Natriumnitrit und 450 ml Schwefelsäure wird auf dem Dampfbad auf ca. 80° erwärmt bis vollständige Lösung eintritt. Nach Abkühlen auf 30° werden 232 g (1,0 Mol) 2-Amino-5-chlorbenzophenon unter Rühren portionenweise zugegeben, so dass die Temperatur zwischen 30 und 40° bleibt. Nach einer Stunde wird die Mischung langsam zu 3 Liter Eiswasser gegeben und über Hy-Flo(R) filtriert. Das Filtrat wird unter Rühren langsam mit einer Lösung von 200 g (1,83 Mol) Natriumtetrafluoroborat in 800 ml Wasser versetzt. Der dabei entstehende Niederschlag wird abfiltriert und 2mal mit je 100 ml Wasser gewaschen.

Das feuchte 2-Benzoyl-4-chlorbenzoldiazoniumtetrafluoroborat wird in 3 Liter Wasser suspendiert und tropfenweise mit einer Lösung von 332 g (2 Mol) Kaliumjodid in 1 Liter Wasser versetzt. Die Mischung wird während 4 Stunden bei Raumtemperatur gerührt; anschliessend filtriert man das Rohprodukt ab und gibt es zu 1 Liter siedendem Äther. Nach Filtrieren und Trocknen über Natriumsulfat wird die ätherische Lösung auf 500 ml eingeengt. Durch Zugeben von 100 ml Petroläther erhält man 5-Chlor-2-jod-benzophenon. Eine Probe des Materials wird aus Äther/Petroläther umkristallisiert und ergibt feine gelbe Prismen vom Smp. 80–82°.

Beispiel 2

Die Herstellung von 5-Chlor-2′-fluor-2-jod-benzophenon erfolgt auf die gleiche Weise wie die Herstellung von 5-Chlor-2-jod-benzophenon und ergibt hellgelbe Prismen vom Smp. 78–81°.

Beispiel 3

Eine Mischung aus 0,17 g (1,0 mMol) Palladiumchlorid, 0,5 g (2,0 mMol) Triphenylphosphin, 0,1 g (0,5 mMol) Kupferjodid, 15 g (43 mMol) 5-Chlor-2-jod-benzophenon und 60 ml Diäthylamin wird bei Raumtemperatur während 20 Minuten gerührt. In einer Portion gibt man 6,0 g (107 mMol) Propargylalkohol hinzu und rührt die entstandene Mischung während 24 Stunden. Nach Entfernen des Lösungsmittels im Vakuum, wird der Rückstand in Äther aufgenommen. Die ätherische Lösung wird mit Wasser gewaschen, über Natriumsulfat getrocknet und im Vakuum eingedampft. Man erhält

3-Hydroxy-1-[4-chlor-2-benzoyl-phenyl]-propin

als rotes Öl.

Beispiel 4

Eine Mischung aus 0,37 g (0,5 mMol) Dichlor-bis-triphenylphosphin-Palladium II, 70 mg (0,35 mMol) Kupfer-jodid, 36,1 g (0,1 Mol) 5-Chlor-2′-fluor-2-jod-benzophenon, 12 ml (0,2 Mol) Propargylalkohol und 200 ml Diäthylamin wird bei Raumtemperatur während 4 Tagen gerührt. Nach Einengen der Mischung im Vakuum wird der Rückstand zwischen Äther und Wasser verteilt. Die ätherische Phase wird mit Wasser gewaschen, über Natriumsulfat getrocknet und im Vakuum eingedampft. Dabei erhält man

3-Hydroxy-1-[4-Chlor-2-(2-fluorbenzoyl)-phenyl]-propin

als gelbbraunes Öl.

Beispiel 5

Zu einer auf 0° gekühlten Lösung von 28,9 g (0,1 Mol)

3-Hydroxy-1-[4-chlor-2-(2-fluorbenzoyl)-phenyl]-propin,

24,4 ml (0,175 Mol) Triäthylamin in 300 ml Methylenchlorid werden 13 ml (0,17 Mol) Methansulfonylchlorid tropfenweise zugegeben. Die Mischung wird nacheinander mit Eiswasser, kalter 1N Salzsäure und kalter gesättigter Natriumbicarbonat-Lösung gewaschen und über Natriumsulfat getrocknet. Durch Eindampfen der organischen Phase erhält man ein braunes Öl, welches aus Äther zu einem gelben Festkörper kristallisiert. Durch Umkristallisation aus Äther/Petroläther erhält man

3-Hydroxy-1-[4-chlor-2-(2-fluorbenzoyl)-phenyl]-propin-methansulfonat

als farblose Prismen vom Smp. 95–96°.

Beispiel 6

Eine Mischung aus 5,8 g (16 mMol)

3-Hydroxy-1-[4-chlor-2-(2-fluorbenzoyl)-phenyl]-propin-methansulfonat

und 50 ml methanolischer Hydroxylamin-Lösung (aus 6,1 g, 88 mMol Hydroxylaminhydrochlorid) in 50 ml Tetrahydrofuran wird bei Raumtemperatur während 13 Stunden gerührt. Die Mischung wird im Vakuum eingedampft, der Rückstand wird zwischen Methylenchlorid und Wasser verteilt. Nach Trocknen der organischen Phase über Natriumsulfat und Eindampfen im Vakuum erhält man ein gelbbraunes Öl, welches

3-Hydroxyamino-1-[4-chlor-2-(2-fluorbenzoyl)-phenyl]-propin

enthält.

Eine Lösung von 3,4 g dieses gelbbraunen Öls in 70 ml Methylenchlorid wird tropfenweise zu einer auf 0 °C gekühlten Mischung aus 0,7 g (2,3 mMol) Quecksilbersulfat und 17 ml Ameisensäure gegeben. Man rührt die Mischung bei Raumtemperatur über Nacht, giesst auf Eis und stellt mit Ammoniumhydroxid basisch. Die Methylenchlorid-Lösung wird mit Wasser gewaschen, über Natriumsulfat getrocknet und im Vakuum eingedampft. Reinigung des so erhaltenen gelben Öls durch Säulenchromatographie (Silicagel, 50 g; 3 : 1 Methylenchlorid/Äther als Elutionsmittel) ergibt braune Kristalle vom Smp. 165–167°. Umkristallisation aus Äther/Methylenchlorid ergab

8-Chlor-1-(2-fluorphenyl)-3,4-dihydro-5H-2-benzazepin-5-on-2-oxid

als schwach gelbe Prismen vom Smp. 167–170°.

Beispiel 7

Eine Mischung aus 3,4 g (11 mMol) 8-Chlor-1-(2-fluor-phenyl)-3,4-dihydro-5H-2-benzazepin-5-on-2-oxid und 26 ml Dimethylformamid-dimethylacetal wird während 12 Stunden bei Raumtemperatur gerührt. Nach Verdünnen der Mischung mit Äther und Filtrieren erhält man einen gelben Festkörper vom Smp. 175–178°. Umkristallisation aus Äther/Essigester ergibt

8-Chlor-1-(2-fluorphenyl)-3,4-dihydro-4-[(dimethylamino)methylen]-5H-2-benzazepin-5-on-2-oxid

als gelbe Nadeln vom Smp. 193–194°.

**Patentansprüche für die Vertragsstaaten BE, CH, DE, FR, GB, IT, LU, NL, SE.**

1. Verbindungen der allgemeinen Formel

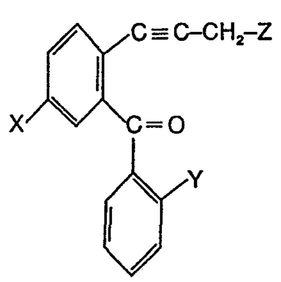

I

worin X und Y je Wasserstoff, Halogen mit einer Atomnummer von höchstens 35 oder Trifluormethyl und

Z Hydroxy, Methansulfonyloxy, Toluolsulfonyloxy, Brom oder Chlor bedeuten.

2. Verbindungen gemäss Anspruch 1, worin Z Hydroxy oder Methansulfonyloxy bedeutet.

3. 3-Hydroxy-1-[4-chlor-2-(2-fluorbenzoyl)-phenyl]-propin.

4. 3-Hydroxy-1-[4-chlor-2-benzoylphenyl]-propin.

5. 3-Hydroxy-1-[4-chlor-2-(2-fluorbenzoyl)-phenyl]-propin-methansulfonat.

6. Verfahren zur Herstellung von Propargylderivaten der in Anspruch 1 definierten allgemeinen Formel I, dadurch gekennzeichnet, dass man eine Verbindung der allgemeinen Formel

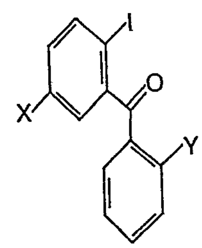

II

worin X und Y die in Anspruch 1 angegebene Bedeutung besitzen,

in Gegenwart eines Palladium-(II)-Salzes, eines Organophosphins, eines tertiären oder sekundären Amins und von Kupfer-(I)-Jodid mit Propargylalkohol zur Reaktion bringt und erwünschtenfalls die erhaltene Verbindung der Formel I, worin Z Hydroxy bedeutet, in eine Verbindung der Formel I, worin Z Methansulfonyloxy, Toluolsulfonyloxy, Brom oder Chlor bedeutet, überführt.

**Patentansprüche für den Vertragsstaat AT**

1. Verfahren zur Herstellung von Propargylderivaten der allgemeinen Formel

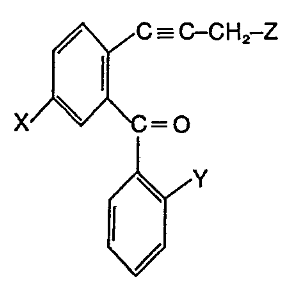

I

worin X und Y je Wasserstoff, Halogen mit einer Atomnummer von höchstens 35 oder Trifluormethyl und

Z Hydroxy, Methansulfonyloxy, Toluolsulfonyloxy, Brom oder Chlor bedeuten,

dadurch gekennzeichnet, dass man eine Verbindung der allgemeinen Formel

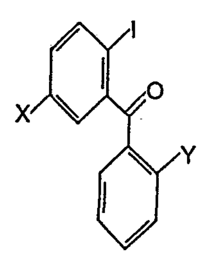

II

worin X und Y obige Bedeutung besitzen,

in Gegenwart eines Palladium-(II)-Salzes, eines Organophosphins, eines tertiären oder sekundären Amins und von Kupfer-(I)-Jodid mit Propargylalkohol zur Reaktion bringt und erwünschtenfalls die erhaltene Verbindung der Formel I, worin Z Hydroxy bedeutet, in eine Verbindung der Formel I, worin Z Methansulfonyloxy, Toluolsulfonyloxy, Brom oder Chlor bedeutet, überführt.

2. Verfahren nach Anspruch 1, dadurch gekennzeichnet, dass man eine Verbindung der Formel I herstellt, worin Z Hydroxy oder Methansulfonyloxy bedeutet.

3. Verfahren nach Anspruch 2, dadurch gekennzeichnet, dass man
3-Hydroxy-1-[4-chlor-2-(2-fluorbenzoyl)-phenyl]-propin
herstellt.

4. Verfahren nach Anspruch 2, dadurch gekennzeichnet, dass man
3-Hydroxy-1-[4-chlor-2-benzoylphenyl]-propin
herstellt.

5. Verfahren nach Anspruch 2, dadurch gekennzeichnet, dass man
3-Hydroxy-1-[4-chlor-2-(2-fluorbenzoyl)-phenyl]-propin-methansulfonat
herstellt.

**Claims for the Contracting States: BE, CH, DE, FR, GB, IT, LU, NL, SE**

1. Compounds of the general formula

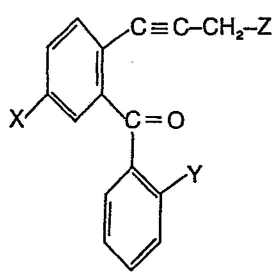

I

wherein X and Y each signify hydrogen, halogen with an atomic number of at most 35 or trifluoromethyl and Z signifies hydroxy, methanesulphonyloxy, toluenesulphonyloxy, bromine or chlorine.

2. Compounds in accordance with claim 1, wherein Z signifies hydroxy or methanesulphonyloxy.

3. 3-Hydroxy-1-[4-chloro-2-(2-fluorobenzoyl)-phenyl]-propyne.

4. 3-Hydroxy-1-[4-chloro-2-benzoylphenyl]-propyne.

5. 3-Hydroxy-1-[4-chloro-2-(2-fluorobenzoyl)-phenyl]-propyne methanesulphonate.

6. A process for the manufacture of propargyl derivatives of general formula I defined in claim 1, characterized by reacting a compound of the general formula

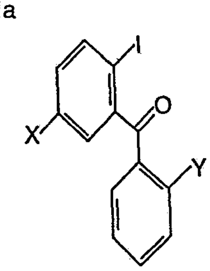

II

wherein X and Y have the significance given in claim 1, with propargyl alcohol in the presence of a palladium (II) salt, an organophosphine, a tertiary or secondary amine and of copper (I) iodide and, if desired, converting the resulting compound of formula I in which Z signifies hydroxy into a compound of formula I in which Z signifies methanesulphonyloxy, toluenesulphonyloxy, bromine or chlorine.

**Claims for the Contracting State: AT**

1. A process for the manufacture of propargyl derivatives of the general formula

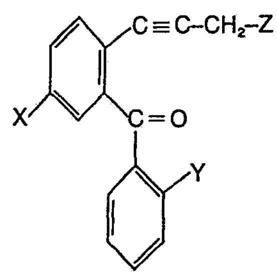

I

wherein X and Y each signify hydrogen, halogen with an atomic number of at most 35 or trifluoromethyl and Z signifies hydroxy, methanesulphonyloxy, toluenesulphonyloxy, bromine or chlorine, characterized by reacting a compound of the general formula

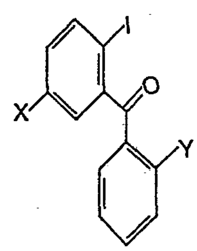

II

wherein X and Y have the above significance, with propargyl alcohol in the presence of a palladium (II) salt, an organophosphine, a tertiary or secondary amine and of copper (I) iodide and, if desired, converting the resulting compound of formula I in which Z signifies hydroxy into a compound of formula I in which Z signifies methanesulphonyloxy, toluenesulphonyloxy, bromine or chlorine.

2. A process according to claim 1, characterized in that a compound of formula I in which Z signifies hydroxy or methanesulphonyloxy is manufactured.

3. A process according to claim 2, characterized in that
3-hydroxy-1-[4-chloro-2-(2-fluorobenzoyl)-phenyl]-propyne
is manufactured.

4. A process according to claim 2, characterized in that
3-hydroxy-1-[4-chloro-2-benzoylphenyl]-propyne
is manufactured.

5. A process according to claim 2, characterized in that
3-hydroxy-1-[4-chloro-2-(2-fluorobenzoyl)-phenyl]-propyne
methanesulphonate is manufactured.

**REVENDICATIONS POUR LES ETATS CONTRACTANTS: BE, CH, DE, FR, GB, IT, LU, NL, SE**

1. Composés de formule générale

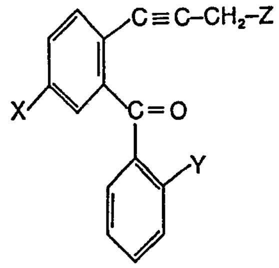

I

dans laquelle X et Y représentent chacun l'hydrogène, un halogène de numéro atomique 35 au maximum ou le groupe trifluorométhyle et
Z représente un groupe hydroxy, méthane-sulfonyloxy, toluène-sulfonyloxy, le brome ou le chlore.
2. Composés selon la revendication 1, dans lesquels Z représente un groupe hydroxy ou méthane-sulfonyloxy.
3. Le 3-hydroxy-1-[4-chloro-2-(2-fluorobenzoyl)-phényl]-propyne.
4. Le 3-hydroxy-1-[4-chloro-2-benzoylphényl]-propyne.
5. Le méthane-sulfonate du 3-hydroxy-1-[4-chloro-2-(2-fluorobenzoyl)-phényl]-propyne.
6. Procédé de préparation des dérivés propargyliques de formule générale I défini dans la revendication 1, caractérisé en ce que l'on fait réagir un composé de formule générale:

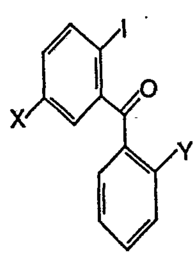

II

dans laquelle X et Y ont les significations indiquées dans la revendication 1, en présence d'un sel de palladium-II, d'une organophosphine, d'une amine tertiaire ou secondaire et d'iodure de cuivre-I avec l'alcool propargylique, après quoi, si on le désire, on convertit le composé obtenu, répondant à la formule I dans laquelle Z représente un groupe hydroxy, en un composé de formule I dans laquelle Z représente un groupe méthane-sulfonyloxy, toluène-sulfonyloxy, le brome ou le chlore.

**REVENDICATIONS POUR L'ETAT CONTRACTANT: AT**

1. Procédé de préparation de dérivés propargyliques de formule générale

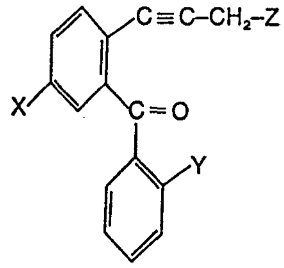

I

dans laquelle X et Y représentent chacun l'hydrogène, un halogène de numéro atomique 35 au maximum ou le groupe trifluorométhyle et
Z représente un groupe hydroxy, méthane-sulfonyloxy, toluène-sulfonyloxy, le brome ou le chlore,
caractérisé en ce que l'on fait réagir un composé de formule générale

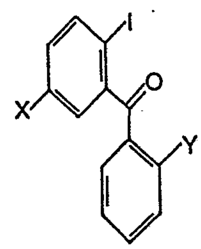

II

dans laquelle X et Y ont les significations indiquées ci-dessus,
en présence d'un sel de palladium-II, d'une organophosphine, d'une amine tertiaire ou secondaire et d'iodure de cuivre-I avec l'alcool propargylique et, si on le désire, on convertit le composé obtenu, répondant à la formule I dans laquelle Z représente un groupe hydroxy, en un composé de formule I dans laquelle Z représente un groupe méthane-sulfonyloxy, toluène-sulfonyloxy, le brome ou le chlore.
2. Procédé selon la revendication 1, caractérisé en ce que l'on prépare un composé de formule I dans laquelle Z représente un groupe hydroxy ou méthane-sulfonyloxy.
3. Procédé selon la revendication 2, caractérisé en ce que l'on prépare le 3-hydroxy-1-[4-chloro-2-(2-fluorobenzoyl)-phényl]-propyne.
4. Procédé selon la revendication 2, caractérisé en ce que l'on prépare le 3-hydroxy-1-[4-chloro-2-benzoylphényl]-propyne.
5. Procédé selon la revendication 2, caractérisé en ce que l'on prépare le méthane-sulfonate du 3-hydroxy-1-[4-chloro-2-(2-fluorobenzoyl]-phényl]-propyne.